# EUROPEAN PATENT SPECIFICATION

(11) **EP 4 059 490 B1**
(45) Date of publication and mention of the grant of the patent: **05.06.2024**
(21) Application number: 22150228.9
(22) Date of filing: 17.03.2021
(51) Int. Cl.: A61K 9/107, A61K 47/22

(54) **PROCESS FOR MANUFACTURING VITAMIN D FORMULATIONS**
VERFAHREN ZUR HERSTELLUNG VON VITAMIN-D-FORMULIERUNGEN
PROCÉDÉ DE FABRICATION DE FORMULATIONS DE VITAMINE D

(43) Date of publication of application: 21.09.2022
(62) Divisional of application: 21163104.9
(73) Proprietor: Solmic Biotech GmbH, 40225 Düsseldorf (DE)
(72) Inventor: FURCH, Marcus, 65719 Hofheim am Taunus (DE); BESECKE, Karen F.W., 30167 Hannover (DE); DINKLER, Helge, 46047 Oberhausen (DE); MACZKA, Michael, 40223 Düsseldorf (DE)
(74) Representative: Schmitz, Joseph

(56) References cited:
- EP-A2- 1 945 185
- EP-B1- 1 945 185
- WO-A1-2014/151109
- WO-A1-2019/175830
- US-A1- 2011 052 707

## Description

### TECHNICAL FIELD

The present invention relates to the manufacture of Vitamin D formulations, in particular pharmaceutical formulations comprising Vitamin D, which can be used in the treatment of, in particular, a Vitamin D deficiencies or PMS.

### PRIOR ART

Vitamin D represents a group of fat-soluble secosteroids, which play a role in intestinal absorption of calcium, magnesium, and phosphate, bone health, cell growth, neuromuscular functions and a balanced immune system. In humans, one of the most important vitamin D species are Vitamin D (calcitriol, the active form), and Vitamin D₃ (cholecalciferol, below) and Vitamin D₂ (ergocalciferol, below), both precursors to calcitriol, which has widespread actions throughout the body by regulating numerous cellular pathways.

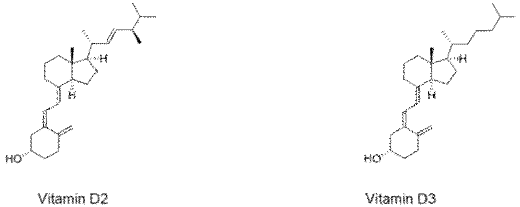

Several epidemiological studies show that in Europe and the USA, a significant percentage of the population (40% to 70%) suffers from low vitamin D blood serum levels, especially during wintertime when sun exposure is minimal in temperate regions of Europe and the USA.

In general, levels of less than 25 nmol/I are found in 10 to 30%, and inadequate levels of 30 to 50 nmol/l are found in more than 50% of the respective study populations. It is to be noted that this is not merely a European or US problem, but that equally populations in Africa and Asia have been found to exhibit insufficient Vitamin D blood serum levels.

Pharmaceutical preparations, as well as nutraceuticals, aimed at addressing deficiencies of Vitamin D in both humans and animal are well-known. Mostly, these preparations are oral dosage forms: either liquid oil-based or solid dosage forms (such as tablets or gel capsules). Such preparations can have limited shelf-life because the Vitamin D and its derivatives are susceptible of photodegradation.

While one could assume that deficiencies of Vitamin D can be easily addressed by oral intake of the aforementioned preparations, this is not quite the case. Uptake of Vitamin D and hence, the bioavailability of the Vitamin D is rather low and thus, high doses of up to 300 0000 IU are generally administered intramuscularly to achieve blood serum levels of more than 30 ng/ml in subjects with deficiency of Vitamin D, i.e. having a blood serum levels of less than 20 ng/ml.

It is thus desirable to solve, or at least alleviate, the above-mentioned problem of limited absorption and bioavailability of Vitamin D seen in existing pharmaceutical or nutraceutical preparations, preferably without having to resort to preparations that comprise high doses of Vitamin D.

### SUMMARY OF THE INVENTION

The above-mentioned problems can be solved by a formulation that can be produced according to the process according to claim 1 and provide a means to remedy vitamin D deficiency in mammals, in particular humans, by achieving required blood serum levels of Vitamin D without reverting to injection forms containing doses of Vitamin D of up to 300 000 lUs.

The thus produced formulation adapted for oral administration, wherein the pharmaceutical formulation comprises at least 1 000 IU, preferably of from 5 000 to 400 000 IU of Vitamin D, Vitamin D₂, Vitamin D₃, or mixtures thereof per dosage form, and wherein the pharmaceutical formulation comprises, or consists of, an aqueous dispersion of micelles encapsulating said Vitamin D, Vitamin D₂, Vitamin D₃, or mixtures thereof and wherein the micelles are formed of, and essentially consist of, a tocopherol polyethylene glycol such as D-α-tocopherol polyethylene glycol succinate.

The formulation may for example be a pharmaceutical formulation, a nutraceutical, or a dietary complement. In particular, the formulation is a pharmaceutical formulation.

The micelles dispersed in the aqueous dispersion have a diameter of less than 50 nm, and preferably a diameter of from 10 to 50 nm, 20 to 50 nm, or 30 to 50 nm and most preferably have a diameter in the range of from 20 to 30 nm.

It is an object of the present invention to provide process for producing a pharmaceutical formulation adapted for oral administration wherein the pharmaceutical formulation comprises at least 1 000 IU of a Vitamin D, per dosage form, and wherein the pharmaceutical formulation comprises, or consists of, an aqueous dispersion of micelles encapsulating said Vitamin D, and wherein the micelles are formed of, and essentially consist of, a tocopherol polyalkylene glycol, said process comprising the steps of
a. combining an amount of a Vitamin D in a molten state with an amount of a tocopherol polyalkylene glycol such as D-α-tocopherol polyethylene glycol succinate in a molten state to form a first melt mixture, wherein preferably the amount of the Vitamin D constitutes of from 1 to 10 weight percent, more preferably of from 1 to 5 weight percent of the first mixture, based on the total amount of tocopherol polyalkylene glycol and Vitamin D,
b. stirring and maintaining a temperature for the first melt mixture to remain in the molten state,
c. combining, while stirring, the first melt mixture with an aqueous solution having an acidic pH, wherein the aqueous solution having an acidic pH has about the same temperature than the first mixture melt, to obtain a second mixture,
d. stirring and heating the second mixture until an aqueous dispersion nanoemulsion of micelles is formed encapsulating the Vitamin D is formed.

The thus produced pharmaceutical formulations may be used as a medicament, in particular in the treatment of a Vitamin D deficiency, by a dosage regimen comprising:
a. administering a loading dose of the pharmaceutical formulation comprising at least 5 000 IU, preferably of from 5 000 to 400 000 IU of a Vitamin D, and more preferably of from 50 000 IU to 100 000 IU, on the first day of a period of one month to six months, preferably of a period of two, three or four months,
b. optionally and subsequently administering a dose comprising less than 5 000 IU, preferably of from 500 or from 1000 to 5 000 IU of a Vitamin D, on the second day of the period of one month to six months, preferably of the period of two, three or four months, and subsequently daily for the remainder of the period of one month to six months, preferably of the period of two, three or four months.

The thus produced pharmaceutical formulations may be used in the treatment of a Vitamin D deficiency, by a dosage regimen comprising:
c. administering a loading dose of the pharmaceutical formulation comprising of from 50 000 IU to 100 000 IU of a Vitamin D, on the first day of a period of two months,
d. optionally and subsequently administering a dose comprising less than 5 000 IU, preferably of from 500 or from 1 000 to 5 000 IU of a Vitamin D, on the second day of the period of two months, and subsequently daily for the remainder of the period of two months, wherein the dosage regimen is repeated three times over a period of six months.

The thus produced formulations may be included in a kit, for use in the treatment of a Vitamin D deficiency, the kit comprising:
a. at least one loading dose of the pharmaceutical formulation according to the above, comprising at least 5 000 IU, preferably of from 5 000 to 400 000 IU of a Vitamin D, more preferably of from 50 000 to 100 000 IU of a Vitamin D,
b. optionally, a plurality of daily doses, each comprising less than 5 000 IU, preferably of from 500 or from 1000 to 5 000 IU of a Vitamin D, wherein the number of daily doses in the plurality of daily doses is such that a daily dose can be administered for a period of one month to six months, preferably at least for a the period of two, three or four months,
c. instructions for a dosage regime consisting of
   i. administering the loading dose on the first day of the period of one month to six months, preferably of the period of two, three or four months and
   ii. administering a daily dose, on the second day of the period of one month to six months, preferably for the period of two, three or four months and thereafter on a daily basis for the remainder of the period of one month to six months, preferably for the period of two, three or four months.

The thus produced formulations may be included in a kit, for use in the treatment of a Vitamin D deficiency, the kit comprising :
a. three loading doses of the pharmaceutical formulation according to the above, comprising of from 50 000 to 100 000 IU of a Vitamin D,
b. optionally, a plurality of daily doses, each comprising of from 500 or from 1000 to 5 000 IU of a Vitamin D, wherein the number of daily doses in the plurality of daily doses is such that a daily dose can be administered for a period of six months,
c. instructions for a dosage regime consisting of
   i. administering a loading dose on the first day of the first month of the period of six months, and then on the first day of every other month of the period of six months,
   ii. administering a daily dose, on the second day of the period of six months, and thereafter on a daily basis for the remainder of the period of six months, except on the days a loading dose is administered.

Further embodiments of the invention are laid down in the dependent claims.

### DESCRIPTION OF PREFERRED EMBODIMENTS

A pharmaceutical formulation adapted for oral administration may be produced according to the process of claim 1, wherein said pharmaceutical formulation comprises at least 1 000 IU, preferably of from 5 000 to 400 000 IU of a Vitamin D, per dosage form, and wherein the pharmaceutical formulation comprises, or consists of, an aqueous dispersion of micelles encapsulating said Vitamin D, and wherein the micelles are formed of, and essentially consist of, a tocopherol polyalkylene glycol such as D-α-tocopherol polyethylene glycol succinate.

It is understood that in the context of the present invention "a Vitamin D" is to be understood as referring to any of Vitamin D (calcitriol), Vitamin D₂ (ergocalciferol) and Vitamin D₃ (cholecalciferol) or combinations thereof.

The pharmaceutical formulation that may be produced according to the process of claim 1 is water-soluble, in the sense that it can be diluted in aqueous solution without the micelles of the nanoemulsion disintegrating into their constituents and releasing the encapsulated payload, in this case the Vitamin D, into the aqueous solution.

The pharmaceutical formulation may be in solid, semi-solid, or liquid form. Examples of solid pharmaceutical formulations are for example tablets, capsules such as hard or soft gel capsules, lozenges, dragées. Examples of liquid pharmaceutical formulations are for example sirups or gels.

The pharmaceutical formulation comprises at least 5 000 IU, preferably of from 5 000 to 400 000 IU of a Vitamin D, per dosage form.

The pharmaceutical formulation produced according to the process of claim 1 may comprise of from 5 000 to 200 000 IU of a Vitamin D, per dosage form, more preferably of from 25 000 to 200 000 IU of a Vitamin D and most preferably of from 50 000 to 200 000 IU of a Vitamin D and even more preferably of from 50 000 to 100 000 IU of a Vitamin D.

It has been found that, even at comparatively low doses of from 5 000 to 50 000 IU of a Vitamin D, per dosage form, and in particular at a daily dose of 5 000 IU, an increase of blood serum level of 25(OH)D of about 7 ng/ml could be achieved in human patients, which could hardly be achieved via existing oral oil-based or water-based pharmaceutical formulations that contained doses of less than 100 000 IU. Thus, when using a pharmaceutical formulation produced according to the process of claim 1, a daily dose of from 5 000 to 50 000 IU of a Vitamin D already can efficiently be used as a medicament, in particular in the treatment of Vitamin D deficiency, where other formulations cannot.

The pharmaceutical formulation comprises, or consists of, an aqueous dispersion of micelles encapsulating said Vitamin D, wherein the micelles are formed of, and essentially consist of, a tocopherol polyalkylene glycol such as D-α-tocopherol polyethylene glycol succinate. The Vitamin D is encapsulated in the micelles, and thus sequestered from the aqueous continuous phase in which it is insoluble, and thus a high effective solubility can be achieved, in spite of the dispersion being an aqueous dispersion. Without wishing to be held to a particular theory, it is believed that the unexpectedly good bioavailability of Vitamin D in the pharmaceutical formulation of the invention is due to the fact that micelles correspond, to some extent, to the micelles that are otherwise formed in the intestine via biliary salts during absorption of fats. An aqueous dispersion of micelles encapsulating the Vitamin D can be achieved by the process as described further below.

Suitable tocopherol polyalkylene glycols are preferably chosen from tocopherol polyethylene glycols such as D-α-tocopherol polyethylene glycol succinate.

In the context of the present invention, a Vitamin D is preferably Vitamin D₂, Vitamin D₃, or mixtures of both.

In a preferred embodiment of the process according to the present invention, the dosage form of the pharmaceutical formulation is in the form of a liquid, semi-solid or gel, and/or has a volume of from 500 µl to 10 000 µl more preferably of from 500 µl to 1 000 µl in particular about 500 µl.

In a preferred embodiment of the process according to the present invention, the dosage form of the pharmaceutical formulation is in the form of a liquid or gel, has a volume of from 500 µl to 10 000 µl more preferably of from 500 µl to 1 000 µl in particularabout 500 µl and contained in a disposable container.

In a preferred embodiment of the process according to the process according to the present invention, the pharmaceutical formulation achieves a maximum blood serum concentration of 25(OH)D of at least 35, preferably of from 35 to 80 ng/ml , in particular in humans.

It is noted that, in the context of the present invention, blood serum concentrations of 25(OH)D are provided as measured via direct competitive chemiluminescent immunoassay, in particular the Siemens ADVIA Centaur^{®} Vitamin D Total assay.

It is noted that 25(OH)D refers, as the case may be, to either 25(OH)D₂ or 25(OH)D₃ depending on the precursor being Vitamin D₂ or Vitamin D₃.

A pharmaceutical formulation produced according to the process of claim 1 may be used as a medicament, in particular in the treatment of a Vitamin D deficiency, by a dosage regimen comprising:
a. administering a loading dose of the pharmaceutical formulation according to the present invention comprising at least 5 000 IU, preferably of from 5 000 to 400 000 IU of a Vitamin D, on the first day of a period of one month to six months, preferably of a period of two, three or four months,
b. optionally and subsequently administering a dose comprising less than 5 000 IU, preferably of from 500 to 5 000 IU of a Vitamin D, on the second day of the period of one month to six months, preferably of the period of two, three or four months, and subsequently on a daily basis for the remainder of the period of one month to six months, preferably of the period of two, three or four months.

Administering a loading dose of the pharmaceutical formulation comprising at least 5 000 IU, preferably of from 5 000 to 400 000 IU of Vitamin D, on the first day of a period of one month to six months allows to quickly raise the Vitamin D content i.e. the blood serum levels of 25-OHD, from an insufficient level of less than 20 ng/ml, to a level above 20, 25 or 30 ng/ml.

It is noted that administering a loading dose of the pharmaceutical formulation of at least 5 000 IU of a Vitamin D according to the present invention may or may not be followed by subsequently administering a dose comprising less than 5 000 IU of a Vitamin D, since it has been observed that the loading dose itself can in some cases provide a level of 25-OHD above 30 ng/ml for a prolonged period, i.e. for a period of one month to six months.

In the case where solely the loading dose of the pharmaceutical formulation is administered, no further dose of a Vitamin D is administered for the remainder of the period, until it is eventually repeated.

In the case where both the loading dose of the pharmaceutical formulation and then subsequent doses of a Vitamin D are administered, the subsequent doses are in general inferior as to the amount of Vitamin D, and may comprise less than 5 000 IU, preferably of from 500 to 5 000 IU of a Vitamin D.

The subsequent doses of a Vitamin D are administered on the second day of the period of one month to six months, preferably of the period of two, three or four months, and subsequently on a daily basis for the remainder of the period of one month to six months, preferably of the period of two, three or four months. While these doses may also be administered using the formulation as described above, it not required that they must be. The subsequent doses of a Vitamin D may be administered via oil-based solutions of Vitamin D or via water-based dispersions of Vitamin D. In a preferred embodiment, the subsequent doses of a Vitamin D are administered via formulations according to the present invention.

The dosage regimen may be repeated one or more times, or two, or three times per year. For example, a period of six months may be repeated once, a period of four months may be repeated twice, a period of three months may be repeated thrice, a period of two months may be repeated five times, and so forth, per year, as needed.

A kit, preferably for use in the treatment of a Vitamin D deficiency, may incorporate a formulation produced according to claim 1, comprising:
a. at least one loading dose of the pharmaceutical formulation according to the present invention, comprising at least 5 000 IU, preferably of from 50 000 to 100 000 IU of a Vitamin D,
b. a number of daily doses, each comprising less than 5 000 IU, preferably of from 500 to 5 000 IU of a Vitamin D, wherein the number of daily doses is such that a daily dose can be administered for a period of one month to six months, preferably of the period of two, three or four months,
c. instructions for a dosage regime consisting of
   i. administering the loading dose on the first day of the period of one month to six months, preferably of the period of two, three or four months, and
   ii. administering a daily dose, on the second day of the period of one month to six months, preferably of the period of two, three or four months and thereafter on a daily basis for the remainder of the period of one month to six months, preferably of the period of two, three or four months.

The kit may comprise loading doses, daily doses and instructions for repeating one or more times the dosage regime of first administering a loading dose and then subsequent daily doses, for example for carrying out the dosage regime twice, thrice or four times during a year or the period of one to two months.

The formulation produced according to the above can be used in the treatment of premenstrual syndrome, preferably by a dosage regimen comprising administering a dose of the pharmaceutical formulation comprising at least 1 000 IU, preferably of from 1 000 to 10 000 IU of a Vitamin D, daily. The combination of a Vitamin D and Vitamin E has been known to be useful in the treatment for premenstrual syndrome, and as such, the formulation according to the above, due to its high bioavailability, is useful in the treatment of premenstrual syndrome, especially when the tocopherol polyalkylene glycol is D-α-tocopherol polyethylene glycol succinate, since D-α-tocopherol polyethylene glycol succinate exhibits Vitamin E activity.

### EXPERIMENTAL DATA

### Preparation according to the present invention of a pharmaceutical formulation

A pharmaceutical formulation according to the present invention was prepared by combining an about 50 g of Vitamin D3 in a molten state at about 50 °C with about 990 g of D-α-tocopherol polyethylene glycol succinate in a molten state at about 50 °C to form a first melt mixture, under stirring, and maintaining the first melt mixture in the molten state at a bout 50 °C, and subsequently combining, while stirring at about 250 rpm, the first melt mixture with an aqueous solution of 5 g of potassium sorbate and 2.5 g citric acid at about 50 °C in about 3952 g of water to obtain a second mixture, and finally continuing stirring and heating the second mixture until an aqueous dispersion of micelles encapsulating the Vitamin D is formed. The formation of the aqueous dispersion of micelles encapsulating the Vitamin D is confirmed by the formation of a clear, i.e. not cloudy, and transparent gel-like material.

### Animal data

Male Wistar rats, with a mean body weight of appr. 200 g (Charles River, Cologne, Germany) were kept without food, but with free access to water for 1 day before the experiment. Several Vitamin D₃ formulations (aqueous dispersion, oil solution, micellar) were administered by gavage at different doses; blood was taken at distinct time intervals and after 30 mins. blood samples were centrifuged at 10 000 x g for 5 min. The concentration of 25(OH)D₃ in the serum was determined by a chemiluminiscence assay, which was validated in the NIH Office of Dietary Supplements (ODS) Vitamin D Standardization Program (VDSP) in November 2010. (Siemens ADVIA Centaur Vitamin D Assay: https://cdn0.scrvt.com/39b415fb07de4d96 56c7b5 16d8e2d907/1800000001964888/5e8060a74747/ADVIA_Centaur_Vitamin_D_Total _standardization_1800000001964888.pdf.)

The absorption efficiencies, i.e. the bioavailabilty, of the vitamin D3 from the various formulations was determined by the ratio of areas under the curve (AUC) of the 25-(OH)D₃ concentrations.

Table 1 shows relative bioavailabilities of 25(OH)D₃ of vitamin D3 formulations (micellar and oily) compared to a vitamin D3 dispersion (for 24 hrs) in vivo. Values were obtained after a single administration of the respective formulation in rats.

**Table 1**

| | **Micellar Formulation** | **Oily Fomulation** | **Dispersion** |
|---|---|---|---|
| **10.000 IU** | 5,4 | 2,4 | 1 |
| **5.000 IU** | 3,7 | 1,5 | 1 |
| **2.500 IU** | 3,0 | 2,5 | 1 |
| **1.250 IU** | 2,7 | 2,3 | 1 |

As can be seen, the micellar formulation produced according to the present invention exhibits better bioavailability than the oily solution or aqueous dispersion, in particular at doses of 5000 IU or more.

### Human data

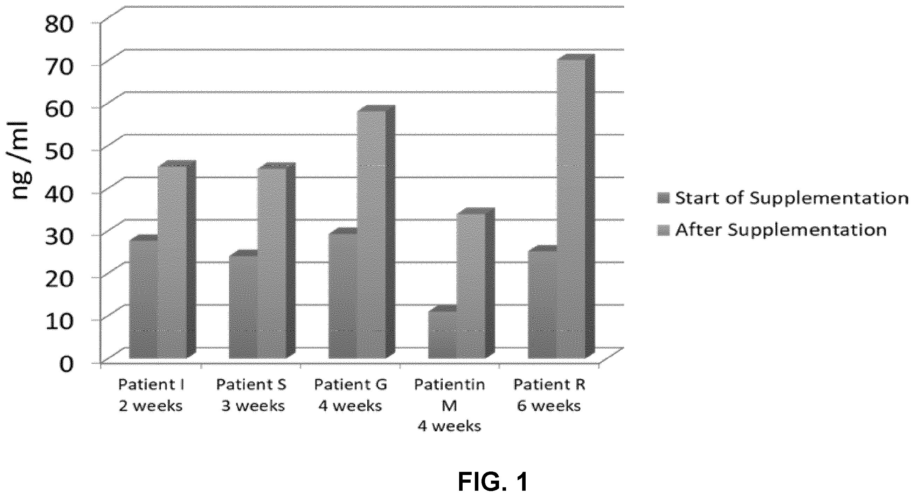

Figure 1 shows the effect of a daily administration of a micellar formulation of Vitamin D₃ according to an embodiment of the present invention in 6 healthy human volunteers with low levels of Vitamin D₃. The effect on the serum level of 25(OH)D₃ of a daily dose of 5 000 IU for up to 6 weeks was determined. For each patient, the left bar shows the serum levels at the start, and the right bar shows the serum levels after 2, 3 4, or 6 weeks of daily administration.

As can be seen in Figure 1, the mean increase of 25(OH)D₃ in the serum was about 7 ng/ml per week, demonstrating that at a daily dose of 5 000 IU, the formulations produced according to the present invention lead to a clear therapeutic benefit.

## Claims

1. A process for producing a formulation adapted for oral administration, wherein the pharmaceutical formulation comprises at least 1 000 IU of a Vitamin D, per dosage form, and wherein the pharmaceutical formulation comprises, or consists of, an aqueous dispersion of micelles encapsulating said Vitamin D, and wherein the micelles are formed of, and essentially consist of, a tocopherol polyalkylene glycol, said process comprising the steps of
a. combining an amount of Vitamin D in a molten state with an amount of tocopherol polyalkylene glycol, such as D-α-tocopherol polyethylene glycol succinate, in a molten state to form a first melt mixture, wherein preferably the amount of Vitamin D constitutes of from 1 to 10 weight percent of the first mixture, based on the total amount of tocopherol polyalkylene glycol and Vitamin D,
b. stirring and maintaining a temperature for the first melt mixture to remain in the molten state,
c. combining, while stirring, the first melt mixture with an aqueous solution having an acidic pH, wherein the aqueous solution having an acidic pH has about the same temperature than the first mixture melt, to obtain a second mixture,
d. stirring and heating the second mixture until an aqueous dispersion of micelles encapsulating the Vitamin D is formed.

2. The process for producing a formulation adapted for oral administration according to claim 1, wherein the pharmaceutical formulation comprises of from 5 000 to 400 000 IU of a Vitamin D, per dosage form.

3. The process for producing a formulation adapted for oral administration according to claim 1 or 2, wherein the micelles are formed of, and essentially consist of, D-α-tocopherol polyethylene glycol succinate.

4. The process for producing a formulation adapted for oral administration according to any preceding claim, wherein the Vitamin D is Vitamin D2, Vitamin D3, or mixtures thereof.

5. The process for producing a formulation adapted for oral administration according to any preceding claim, wherein the dosage form has a volume of about 500 µl to 10 000 µl.

6. The process for producing a formulation adapted for oral administration according to any preceding claim, wherein the micelles have diameter of from 10 nm to 50 nm.

7. The process for producing a formulation adapted for oral administration according to any preceding claim, wherein the pharmaceutical formulation comprises of from 50 000 IU to 100 000 IU of a Vitamin D, per dosage form.

8. The process for producing a formulation adapted for oral administration according to any preceding claim, wherein the amount of Vitamin D constitutes of from 1 to 10 weight percent of the first mixture, based on the total amount of tocopherol polyalkylene glycol and Vitamin D.

9. The process for producing a formulation adapted for oral administration according to any preceding claim, wherein the amount of Vitamin D constitutes of from 1 to 5 weight percent of the first mixture, based on the total amount of tocopherol polyalkylene glycol and Vitamin D.

## Patentansprüche

1. Verfahren zur Herstellung einer zur oralen Verabreichung angepassten Formulierung, wobei die pharmazeutische Formulierung mindestens 1000 Internationale Einheiten (IU) eines Vitamin D pro Dosierungsform enthält, und wobei die pharmazeutische Formulierung eine wässrige Dispersion von Mizellen, welche das besagte Vitamin D einkapseln, enthält oder daraus besteht, und wobei die Mizellen aus einem Tocopherol-Polyalkylenglykol gebildet sind und im Wesentlichen daraus bestehen, wobei das besagte Verfahren die folgenden Schritte beinhaltet:
a. Kombinieren einer Menge von Vitamin D in einem geschmolzenen Zustand, mit einer Menge an Tocopherol-Polyalkylenglykol, wie beispielsweise D-α-Tocopherol-Polyethylenglykol-Succinat, in einem geschmolzenen Zustand, um eine erste Schmelzmischung herzustellen, wobei vorzugsweise die Menge an Vitamin D aus von 1 bis 10 Gewichtsprozent der ersten Mischung besteht, basierend auf der Gesamtmenge an Tocopherol-Polyalkylenglykol und Vitamin D,
b. Rühren und Beibehalten einer Temperatur für die erste Schmelzmischung, um in dem geschmolzenen Zustand zu verbleiben,
c. Kombinieren, während des Rührens, der ersten Schmelzmischung mit einer wässrigen Lösung, welche einen sauren pH-Wert aufweist, wobei die wässrige Lösung, welche einen sauren pH-Wert aufweist, etwa die gleiche Temperatur aufweist, wie die erste Schmelzmischung, um eine zweite Mischung zu erzeugen,
d. Rühren und Erhitzen der zweiten Mischung, bis eine wässrige Dispersion von Mizellen, welche das Vitamin D einkapseln, gebildet wird.

2. Verfahren zur Herstellung einer zur oralen Verabreichung angepassten Formulierung gemäss Anspruch 1, wobei die pharmazeutische Formulierung von 500 bis 400'000 IU eines Vitamin D pro Dosierungsform enthält.

3. Verfahren zur Herstellung einer zur oralen Verabreichung angepassten Formulierung gemäss Anspruch 1 oder 2, wobei die Mizellen aus D-α-Tocopherol-Polyethylenglykol-Succinat gebildet sind und im Wesentlichen daraus bestehen.

4. Verfahren zur Herstellung einer zur oralen Verabreichung angepassten Formulierung gemäss einem der vorhergehenden Ansprüche, wobei es sich bei dem Vitamin D um Vitamin D2, Vitamin D3, oder Mischungen davon handelt.

5. Verfahren zur Herstellung einer zur oralen Verabreichung angepassten Formulierung gemäss einem der vorhergehenden Ansprüche, wobei die Dosierungsform ein Volumen von etwa 500 µl bis 10'000 µl aufweist.

6. Verfahren zur Herstellung einer zur oralen Verabreichung angepassten Formulierung gemäss einem der vorhergehenden Ansprüche, wobei die Mizellen einen Durchmesser von 10 nm bis 50 nm aufweisen.

7. Verfahren zur Herstellung einer zur oralen Verabreichung angepassten Formulierung gemäss einem der vorhergehenden Ansprüche, wobei die pharmazeutische Formulierung von 50'000 IU bis 100'000 IU eines Vitamin D pro Dosierungsform enthält.

8. Verfahren zur Herstellung einer zur oralen Verabreichung angepassten Formulierung gemäss einem der vorhergehenden Ansprüche, wobei die Menge an Vitamin D von 1 bis 10 Gewichtsprozent der ersten Mischung beträgt, basierend auf der Gesamtmenge an Tocopherol-Polyalkylenglykol und Vitamin D.

9. Verfahren zur Herstellung einer zur oralen Verabreichung angepassten Formulierung gemäss einem der vorhergehenden Ansprüche, wobei die Menge an Vitamin D von 1 bis 5 Gewichtsprozent der ersten Mischung beträgt, basierend auf der Gesamtmenge von Tocopherol-Polyalkylenglykol und Vitamin D.

## Revendications

1. Un procédé de production d'une formulation adaptée pour une administration orale, dans lequel la formulation pharmaceutique comprend au moins 1 000 UI d'une vitamine D, par forme posologique, et dans lequel la formulation pharmaceutique comprend, ou est constituée d'une dispersion aqueuse de micelles encapsulant ladite vitamine D, et dans lequel les micelles sont formées et essentiellement constituées d'un tocophérol polyalkylène glycol, ledit procédé comprenant les étapes consistant à :
a. combiner une quantité de vitamine D à l'état fondu avec une quantité de tocophérol polyalkylène glycol, tel que le succinate de D-α-tocophérol polyéthylène glycol, à l'état fondu pour former un premier mélange fondu, dans lequel, de préférence, la quantité de vitamine D constitue de 1 à 10 pour cent en poids du premier mélange, sur la base de la quantité totale de tocophérol polyalkylène glycol et de vitamine D,
b. agiter et maintenir une température pour que le premier mélange fondu reste à l'état fondu,
c. combiner, sous agitation, le premier mélange fondu avec une solution aqueuse ayant un pH acide, la solution aqueuse ayant un pH acide ayant environ la même température que le premier mélange fondu, pour obtenir un deuxième mélange,
d. agiter et chauffer le deuxième mélange jusqu'à formation d'une dispersion aqueuse de micelles encapsulant la vitamine D.

2. Le procédé de production d'une formulation adaptée pour une administration orale selon la revendication 1, dans lequel la formulation pharmaceutique comprend de 5 000 à 400 000 UI de vitamine D, par forme posologique.

3. Le procédé de production d'une formulation adaptée pour une administration orale selon la revendication 1 ou 2, dans lequel les micelles sont formées et sont essentiellement constituées succinate de D-α-tocophérol polyéthylène glycol.

4. Le procédé de production d'une formulation adaptée pour une administration orale selon l'une quelconque des revendications précédentes, dans lequel la vitamine D est la vitamine D2, la vitamine D3 ou des mélanges de celles-ci.

5. Le procédé de production d'une formulation adaptée pour une administration orale selon l'une quelconque des revendications précédentes, dans lequel la forme posologique a un volume d'environ 500 µl à 10 000 µl.

6. Le procédé de production d'une formulation adaptée pour une administration orale selon l'une quelconque des revendications précédentes, dans lequel les micelles ont un diamètre de 10 nm à 50 nm.

7. Le procédé de production d'une formulation adaptée pour une administration orale selon l'une quelconque des revendications précédentes, dans lequel la formulation pharmaceutique comprend de 50 000 UI à 100 000 UI de vitamine D, par forme posologique.

8. Le procédé de production d'une formulation adaptée pour une administration orale selon l'une quelconque des revendications précédentes, dans lequel la quantité de vitamine D constitue de 1 à 10 pour cent en poids du premier mélange, sur la base de la quantité totale de tocophérol polyalkylène glycol et de vitamine D.

9. Le procédé de production d'une formulation adaptée pour une administration orale selon l'une quelconque des revendications précédentes, dans lequel la quantité de vitamine D constitue de 1 à 5 pour cent en poids du premier mélange, sur la base de la quantité totale de tocophérol polyalkylène glycol et de vitamine D.
